# EUROPÄISCHE PATENTSCHRIFT

(11) **EP 0 941 137 B1**
(45) Veröffentlichungstag und Bekanntmachung des Hinweises auf die Patenterteilung: **08.08.2001**
(21) Anmeldenummer: 97909332.5
(22) Anmeldetag: 29.09.1997
(51) Int. Cl.: A62D 3/00

(54) **ABBAU HALOGENIERTER KOHLENWASSERSTOFFE DURCH HALOPHILE ARCHAEA**
DECOMPOSITION OF HALOGENATED HYDROCARBONS BY HALOPHILIC ARCHAE BACTERIA
DECOMPOSITION D'HYDROCARBURES HALOGENES PAR DES ARCHEOBACTERIES HALOPHILES

(30) Priorität: 27.09.1996 DE 19639894
(43) Veröffentlichungstag der Anmeldung: 15.09.1999
(73) Patentinhaber: Patzelt, Heiko, 81543 München (DE)
(72) Erfinder: PATZELT, Heiko, D-81543 München (DE); KESSLER, Brigitte, D-80689 München (DE); OESTERHELT, Dieter, D-81377 München (DE)
(74) Vertreter: Weiss, Wolfgang, Dipl.-Chem. Dr.
(86) Internationale Anmeldenummer: EP9705345
(87) Internationale Veröffentlichungsnummer: WO9813105

(56) Entgegenhaltungen:
- LORENZ ADRAIN ET AL.: "anaerobic degradation of trichlorobenzenes in hihgly enriched mixed cultures" BIOLOGISHE ABWASSERREINIGUNG, 6 MICROBIELLE ELIMINIERUNG CHLORORGANISHER VERBINDUNGEN, Nr. 6, 1995, BERLIN GERMANY, Seite 81-89 XP002050120
- VENTOSA A : "biotechnological applications and potentialities of halophilic microorganisms" WORLD JOURNAL OF MICROBIOLOGY AND BIOTECHNOLOGY, Bd. 11, Nr. 1, Januar 1995, OXFORD NEW YORK, Seiten 85-94, XP002050121

## Beschreibung

Die Erfindung betrifft ein Verfahren zum zumindest partiellen Abbau von halogenierten organischen Verbindungen durch halophile Archaea, Extrakte oder Bestandteile davon. Weiterhin werden Verfahren zur Gewinnung von an halogenierte Verbindungen adaptierten Organismen sowie die durch das Verfahren erhältlichen Organismen offenbart.

Halogenierte Kohlenwasserstoffe, insbesondere Rückstände aus Pflanzenschutzmitteln und Industrieabfällen, stellen wegen ihrer Toxizität und Persistenz ein weltweit wachsendes Umweltproblem dar. Die herkömmlichen Entsorgungsmethoden beinhalten sehr aufwendige Bodenextraktionen und die Endlagerung der versiegelten Giftstoffe auf Deponien bzw. die Verbrennung in speziellen Hochtemperaturöfen. Das Problem bei der Endlagerung auf Deponien besteht insbesondere darin, daß zur Zeit völlig unklar ist, ob eine dauerhafte Versiegelung der Giftstoffe wirklich gewährleistet werden kann. Bei der Verbrennung unter nicht optimalen Bedingungen werden extrem giftige Substanzen, wie Dioxine und Dibenzofurane gebildet.

Über die letzten 15 Jahre hin wurde deshalb eine Reihe von Mikroorganismen auf ihre Fähigkeit untersucht, chlorierte Kohlenwasserstoffe biologisch abzubauen (vgl. den Übersichtsartikel von Fetzner und Lingens, Micorbiological Rewievs 58 (1994), 641-685). Die untersuchten Mikroorganismen, bei denen es sich im allgemeinen um bakterielle Organismen aus den Gattungen Pseudomonas, Alcaligenes, Flavobacterium, Rhodococcus, Streptomyces, Hyphomicrobium und Methylobacterium handelt, transformieren die jeweiligen halogenierten Kohlenwasserstoffe nur mit geringer Effizienz, in geringen Konzentrationen und meist unvollständig.

L.Adrian et al. ("Anaerober Abbau von Trichlorbenzolen in hochangereicherten Mischkulturen", BIOLOGISCHE ABWASSERREINIGUNG Vol. 6, (1995), 81-89), beschreiben ein Verfahren zum mikrobiologischen Abbau von halogenierten organischen Verbindungen mittels Mischkulturen, welche Archaebakterien enthalten. Adrian et al. lehren außerdem, daß methanogene Archaebakterien der Mischkultur an der Dechlorierung nicht beteiligt sind.

Es besteht somit ein großes Bedürfnis, Verfahren zum Abbau von halogenierten organischen Verbindungen bereitzustellen, bei denen die Nachteile des Standes der Technik mindestens teilweise beseitigt sind..Insbesondere soll das Verfahren einen möglichst vollständigen Abbau zu nicht toxischen Endprodukten ermöglichen.

Überraschenderweise wurde festgestellt, daß halophile Archaea an halogenierte organische Verbindungen in bis zu millimolaren Konzentrationen angepaßt werden können. Die Organismen sind in der Lage, die Halogenatome der halogenierten Verbindungen ohne Anreicherung von toxischen Metaboliten, vorzugsweise zu anorganischen Verbindungen wie etwa Mineralsalzen abzubauen. Dieser Abbau konnte exemplarisch an mehreren isomeren Trichlorphenolen, Lindan und DDT in Flüssigkultur durch eine kombinierte gaschromatographisch-massenspektroskopische Analyse der organischen Extrakte aus Fermentationsbrühen belegt werden. Für jeden Abbauweg konnte jeweils mindestens ein Intermediat massenspektroskopisch identifiziert werden.

Der Abbau von halogenierten organischen Verbindungen kann durch vollständige Zellen, Zellextrakte, Zellfraktionen oder isolierte Zellbestandteile wie etwa Enzyme erfolgen. Er kann auch unter Hochsalzbedingungen oder in Gegenwart von organischen Lösungsmitteln stattfinden.

Der erfindungsgemäße Abbau von halogenierten organischen Verbindungen durch halophile Archaea stellt eine wesentliche Verbesserung bestehender Methoden dar. Die hohe Toleranz der halophilen Archaea gegenüber hohen Konzentrationen an halogenierten organischen Verbindungen sowie die Fähigkeit, diese Verbindungen abzubauen, ist angesichts der bekannten Empfindlichkeit von Halobakterien gegenüber Fremdsubstanzen wie Gallensäuren und Antibiotika in höchstem Maße überraschend (vgl. z. B. Kamekura et al., Appl. Enyiron. Microbiol. 54 (1988), 990-995 und Oren, Arch. Microbiol. 165 (1996), 354-358).

Ein Gegenstand der vorliegenden Erfindung ist somit ein Verfahren zum zumindest partiellen Abbau von halogenierten organischen Verbindungen, welches dadurch gekennzeichnet ist, daß man die Verbindungen mit halophilen Archaea oder mit Extrakten oder Bestandteilen davon in Kontakt bringt und unter Bedingungen inkubiert, bei denen ein Abbau der organischen Verbindungen stattfindet.

Halophile Archaea sind eine Unterklasse der Euryarchaeota (vgl. z. B. Woese, Microbiological Rev. 58 (1994), 1-9). Vorzugsweise sind unter dieser Bezeichnung Organismen aus der Ordnung Halobacteriales und besonders bevorzugt Organismen aus der Familie Halobacteriaceae zu verstehen. Beispiele für geeignete Gattungen sind Haloarcula, Halobacterium, Halococcus, Haloferax, Halorubrum, Natronobacterium und Natronococcus. Besonders bevorzugt werden Organismen der Gattungen Haloarcula, Halobacterium und Haloferax verwendet.

Beispiele für geeignete Spezies aus der Gattung Haloarcula sind H.hispanica, H.japonica, H.marismortui, H.vallismortis, H.californiae und H.sinaiiensis. Geeignete Spezies aus der Gattung Halobacterium sind H.salinarium, H.cutirubrum, H.trapanicum, H.sodomense, H.lacusprofundis, H.distributum und Amoebobacter morrhuae (Torreblanca, System. Appl. Microbiol. 8 (1986), 89-99). Geeignete Spezies aus der Gattung Halococcus sind H.morrhuae, H.turkmenicus und H.saccharolyticus. Geeignete Spezies aus der Gattung Haloferax sind H.denitrificans, H.mediterranei, H.gibbonsii und H.volcanii. Eine geeignete Spezies aus der Gattung Halorubrum ist H.saccharovorum. Geeignete Spezies aus der Gattung Natronobacterium sind N.gregoryi, N.magadii und N.pharaonis. Eine geeignete Spezies aus der Gattung Natronococcus ist N.occultus. Bezüglich der Klassifzierung der Organismen wird - sofern nichts anderes angegeben ist - auf den Übersichtsartikel von Tindall (The Procaryonts, Vol 1 (1991), 768-808) oder die Klassifizierung der DSMZ (on-line Katalog: http://www.gbf-braunschweig.de/DSMZ/species/ archaea.htm) verwiesen.

Obwohl zahlreiche natürlich vorkommende Stämme von halophilen Archaea zum Abbau halogenierter organischer Substanzen geeignet sind, werden vorzugsweise solche Organismen verwendet, die einem zusätzlichen Selektionsprozeß zur Adaption an halogenierte organische Verbindungen unterworfen worden sind. Hierzu wird zunächst ein natürlich vorkommender Organismus ausgewählt, der gegenüber bestimmten halogenierten Substanzen wie Chlorphenol oder Lindan, z. B. in Konzentrationen von 1 *µ*M, 10 *µ*M oder 100 *µ*M, Wachstum zeigt. Dieser Stamm wird als Ausgangsmaterial für einen Adaptionsprozeß verwendet, der beispielsweise durch Ausplattierung in Gegenwart steigender Konzentrationen von Giftstoffen wie Lindan oder Chlorbenzol erfolgen kann.

Durch einen solchen Adaptionsprozeß erhält man einen Organismus, der gegenüber dem natürlichen Ausgangsorganismus eine wesentlich höhere Toleranz und Abbauleistung gegenüber halogenierten organischen Substanzen zeigt. Ein Beispiel für einen solchen adaptierten Organismus ist der Stammm Halobacterium CB1, der am 10.09.1996 bei der Deutschen Sammlung von Mikroorganismen und Zellkulturen GmbH, Mascheroder Weg 1b, 38124 Braunschweig, gemäß den Vorschriften des Budapester Vertrags unter dem Aktenzeichen DSM 11147 hinterlegt worden ist. Ein solcher adaptierter Organismus kann anschließend einem weiteren Selektionsprozeß unterzogen werden, bei dem die Toleranz gegenüber speziellen Verbindungen, wie Trichlorphenolen, DDT oder Lindan, nochmals erhöht wird. Der auf diese Weise erhaltene Abkömmling des adaptierten Ausgangsorganismus kann spezifisch dann zum Abbau dieser Verbindungen eingesetzt werden.

Weitere Beispiele für geeignete adaptierte Organismen sind der Stamm Hf-Xl, der aus einem Stamm der Art Haloferax mediterranei hervorgegangen ist und am 24.09.1997 bei der DSMZ unter dem Aktenzeichen DSM 11795 hinterlegt wurde, und der Stamm Hc-U2, der aus einem Stamm der Art Haloarcula californiae hervorgegangen ist und am 24.09.1997 bei der DSMZ unter dem Aktenzeichen DSM 11794 hinterlegt wurde. Hf-X1 eignet sich besonders zum Abbau hochchlorierter Phenole, z.B. Tri-, Tetra und Pentachlorphenole. Hc-U2 eignet sich besonders zum Abbau bromierter Kohlenwasserstoffe wie etwa dem Flammschutzmittel Bis-2,2-(3,5-dibrom-hydroxyphenyl)-propan.

Durch Auswahl geeigneter natürlicher Organismen, wie zuvor angegeben, oder/und durch Anwendung der zuvor beschriebenen Selektionsprozesse können Organismen bereitgestellt werden, die eine Toleranz gegenüber der abzubauenden Verbindung bzw. den abzubauenden Verbindungen in einer Konzentration von mindestens 10 *µ* M, vorzugsweise mindestens 50 *µ*M, besonders bevorzugt mindestens 100 *µ*M und am meisten bevorzugt mindestens 1 mM aufweisen.

Die abzubauenden Verbindungen werden vorzugsweise ausgewählt aus organischen Verbindungen, die Kohlenstoff, gegebenenfalls Wasserstoff und Heteroatome, wie etwa O, N, S und P, sowie mindestens ein Halogenatom enthalten. Vorzugsweise enthalten die Verbindungen mindestens ein Fluor-, Jod-, Brom- oder/und Chloratom, besonders bevorzugt mindestens ein Fluor-, Brom- oder/und Chloratom. Durch das erfindungsgemäße Abbauverfahren werden organisch gebundene Halogenatome in anorganische Halogenide überführt.

Die Halogenatome können an die verschiedensten Arten von Kohlenstoffatomen gebunden sein, z. B. aliphatische (linear oder zyklisch), aromatische oder/und olefinische Kohlenstoffatome. Besonders bevorzugt sind die abzubauenden Verbindungen aus gegebenenfalls mehrfach chlorierten aromatischen Verbindungen, wie etwa chlorierten Phenolen oder Benzolen oder aromatischen Dioxinen oder Furanen wie etwa Dibenzodioxinen oder Dibenzofuranen, z. B. 2,3,4-Trichlorphenol, 2,3,5-Trichlorphenol, 2,4,5-Trichlorphenol, 2-Chlordibenzodioxin (2-MCDD) oder 1,2,3,4-Tetrachlordibenzodioxin (1,2,3,4-TCDD) ausgewählt. Andere Beispiele für geeignete Verbindungen enthalten mehrere Halogenatome, die an aliphatische oder olefinische Kohlenstoffatome und an aromatische Kohlenstoffatome gebunden sind, z. B. DDT oder dessen Abbauprodukt DDE. Noch eine weitere bevorzugte Klasse von abzubauenden Verbindungen sind mehrfach chlorierte alicyclische Verbindungen, wie etwa Lindan (γ-Hexachlorcyclohexan).

Weitere spezifische Beispiele für abzubauende Verbindungen sind 2,3,6-Trichlorphenol, 2,4,6-Trihalogenphenol (wobei Halogen = Fluor, Chlor, Brom, Jod), Chlorbenzol, Benzylchlorid, Phenylethylchlorid, Hexachlorcyklohexane, z.B. α-HCH, β-HCH, γ-HCH und δ-HCH, 2,3,4-Trifluorphenol, 2,3,5-Trifluorphenol, 2,2-Bis-(4-chlorphenyl)-1,1-Dichlorethan (DDD), Chlorparaffinfraktionen, Decabromdiphenylether, Pentahalogenphenol (wobei Halogen = Chlor, Fluor, Brom), Bis-2,2-(3,5-dibrom-4-hydroxyphenyl)-propan, 3,4,5-Trichlorphenol, 2,3,4,5-Tetrachlorphenol, 2,3,4,6-Tetrachlorphenol, Atrazin, Aldrin, Dieldrin, Endrin und Derivate davon wie etwa Endrin-Aldehyd oder Endrin-Keton, Endosulfan I und II, Endosulfan-Sulfat, Heptachlor, Heptachlorepoxid und Methoxychlor sowie Mischungen, die mehrere der genannten Verbindungen enthalten.

Bei besonders lipophilen Giftstoffen wie etwa 2-MCDD, 1,2,3,4-TCDD oder Decabromdiphenylether kann durch Zugabe lösungsvermittelnder Agenzien die Bioverfügbarkeit drastisch erhöht und der Abbau stark beschleunigt werden. Beispiele für solche Agenzien sind Cyclodextrine wie etwa β- oder γ-Cyclodextrine, z.B. Heptakis-2,6-di-O-methyl-β-cyclodextrin. Ebenfalls können als lösungsvermittelnde Agenzien oberflächenaktive Substanzen, z.B. vom Typ der Alkylglykoside oder Cholate verwendet werden. Die lösungsvermittelnden Agenzien werden vorzugsweise in einer Menge von mindestens 0,01 Moläquivalent, besonders bevorzugt von 0,1 -1 Moläquivalent bezogen auf ein Moläquivalent des Giftstoffs der wachsenden Kultur zugesetzt. Besonders tolerant gegenüber höheren Konzentrationen solcher lösungsvermittelnden Agenzien sind z.B. Stämme, die aus Organismen der Gattung Haloarcula, insbesondere H.hispanica, oder Haloferax, insbesondere Haloferox gibbonsii hervorgegangen sind.

Der erfindungsgemäße Abbau kann unter aeroben, mikroaeroben oder anaeroben Bedingungen erfolgen. Unter mikoraeroben Bedingungen ist eine Fermentation in einem versiegelten Reaktionsgefäß zu verstehen, dessen Luftraum nicht entgast wurde. Zu Beginn der Fermentation steht Sauerstoff zur Verfügung, nach einigen Stunden aber fast keiner mehr. Das Wachstum der Organismen kann unter diesen mikroaeroben Bedingungen je nach Stamm durch Zugabe von Arginin oder/und Nitrat verbessert werden. Besonders bevorzugt für den mikroaeroben Abbau sind Stämme, die noch über photosynthetisch aktives Bakteriorhodopsin verfügen, z.B. der Stamm CB1. Hier können bei gleichzeitiger Bestrahlung mit Licht ähnlich hohe Zelldichten wie unter aeroben Fermentationsbedingungen erreicht werden. Bei bestimmten Giftstoffen, z.B. Pentachlorphenol, kann die Toleranz und die Abbaurate auf diese Weise um mindestens eine Größenordnung erhöht werden.

Weiterhin kann die Raum-Zeit-Ausbeute der Abbaureaktioneninsbesondere bei sehr toxischen Substanzen - durch Pulsfütterung oder Zugabe der Substanz bei einer bereits hohen Zelldichte verbessert werden.

Ein weiterer Gegenstand der vorliegenden Erfindung ist ein Verfahren zur Herstellung von an halogenierte organische Verbindungen adaptierten halophilen Archaea-Organismen. Hierbei wählt man einen halophilen Archaea-Ausgangsorganismus aus, der bereits eine Toleranz gegenüber einer vorbestimmten Konzentration einer oder mehrerer ausgewählter halogenierter organischer Verbindungen aufweist, und unterwirft diesen Ausgangsorganismus einem Selektionsprozeß, wobei ein adaptierter Organismus erhalten wird, der gegenüber dem Ausgangsorganismus eine erhöhte Toleranz oder/und Abbauleistung bezüglich den ausgewählten Verbindungen aufweist. Dieser Selektionsprozeß umfaßt vorzugsweise das Ausplattieren des Organismus auf einem festen Kulturmedium in Gegenwart von Giftstoffen, das Identifizieren von Organismen, die bei der jeweiligen Giftstoffkonzentration noch wachsen und gegebenenfalls ein ein- oder mehrmaliges Wiederholen dieser Prozedur bei steigenden Giftstoffkonzentrationen. Als Giftstoffe für diese Primärselektion können z. B. Lindan oder Chlorphenol verwendet werden.

Beispiele für adaptierte Organismen, die auf diese Weise erhalten werden können, sind Halobacterium CB1 (DSM 11147), Haloferax Hf-X1 (DSM 11795), Haloarcula Hc-U2 (DSM 11794) oder Organismen mit einer mindestens gleichen Toleranz oder/und Abbauleistung gegenüber ausgewählten halogenierten organischen Verbindungen, wie etwa Trichlorphenolen, DDT oder Lindan. Den adaptierten Organismus kann man anschließend einem weiteren Selektionsprozeß zur nochmaligen Erhöhung der Toleranz oder/ und Abbauleistung bezüglich spezieller halogenierter organischer Verbindung unterwerfen, wobei man vorzugsweise den Organismus in Gegenwart einer bestimmten Konzentration einer speziellen halogenierten organischen Verbindung kultiviert und diese Kultivierung überlebende Zellen einem oder mehreren weiteren Kultivierungsschritten bei einer höheren Konzentration der Verbindung unterzieht. Dieser Sekundärselektionsprozeß wird vorzugsweise in einem flüssigen Kulturmedium durchgeführt.

Noch ein weiterer Gegenstand der vorliegenden Erfindung ist ein halophiler Archaea-Organismus, der an halogenierte organische Verbindungen adaptiert und durch das zuvor beschriebene Selektionsverfahren erhältlich ist.

Das erfindungsgemäße Verfahren zum Abbau der halogenierten organischen Verbindungen kann sowohl mit vollständigen Zellen als auch mit Extrakten oder Bestandteilen dieser Zellen, z. B. Enzympräparationen, durchgeführt werden. Die Präparation, Isolierung oder Anreicherung von Extrakten oder Enzymen aus halophilen Archaea ist beispielsweise bei Zhang und Poulter, J. Org. Chem. 58 (1993), 3919-3922, Kerscher und Oesterhelt, J. Biochem. 116 (1981), 587-594 und S. DasSarma und E.M. Fleischmann (Hrsg.) Archaea: A laboratory manual. Halophiles. xvi ö 280 p. Cold Spring Harbor Laboratory Press, Plainview, New York, USA, 1995 und Ryu et al., Enzyme Microb. Technol. 16 (1994), 266-275) beschrieben. Der Fachmann kann zur Herstellung von Extrakten, Enzympräparationen oder Enzymen auf die in diesen Publikationen beschriebenen allgemeinen Methoden zurückgreifen.

Die Zellen, Extrakte oder Bestandteile davon können beim erfindungsgemäßen Abbauverfahren sowohl in gelöster Form, aber auch in immobilisierter Form eingesetzt werden. Weiterhin ist es möglich, Zellen, Extrakte und Bestandteile von mehreren Organismen zu kombinieren. Diese Vorgehensweise dürfte insbesondere dann zu empfehlen sein, wenn mehrere halogenierte Verbindungen gleichzeitig vorhanden sind.

Der Abbau wird vorzugsweise unter solchen Bedingungen durchgeführt, bei denen die Zellen, Extrakte oder Bestandteile davon ausreichende Abbauleistungen zeigen. Der Abbau kann in wässrigen Medien, organischen Medien oder Mischungen davon erfolgen. Die Abbautemperatur kann über einen breiten Bereich variiert werden, z. B. 20-60°C. Weiterhin ist bevorzugt, wenn der Abbau unter aeroben Bedingungen, z. B. mit Belüftung durchgeführt wird.

Beispielsweise kann der Abbau in einem Bioreaktor durchgeführt werden, welcher die Zellen, Zellextrakte oder Zellbestandteile in immobilisierter Form enthält. In diesen Bioreaktor werden die abzubauenden organischen Verbindungen in einem Abwasserstrom eingeleitet und im Reaktor für eine ausreichende Zeit gehalten, um den gewünschten Abbaugrad zu erreichen. Der aus dem Bioreaktor abgeleitete Abwasserstrom, der vorzugsweise im wesentlichen frei von organisch gebundenen Halogenatomen ist, kann anschließend entsorgt und beispielsweise in eine Kläranlage eingeleitet werden.

Weiterhin soll die vorliegende Erfindung durch die nachstehenden Beispiele erläutert werden.

### Beispiel 1

### Herstellung von adaptierten Mikroorganismen

10 Isolate der Gattung Halobacterium H.trapanicum, H.salinarium und H.cutirubrum wurden durch Inkubation auf Agarplatten in Gegenwart von Chlorbenzol oder Lindan mit oder ohne Belichtung in Konzentrationen von 0,1 *µ*M - 10 mM auf ihre Toleranz gegenüber halogenierten organischen Verbindungen getestet. 4 der 10 getesteten Stämme, von denen jeweils 2 aus H.salinarium und H.trapanicum Isolaten hervorgingen, konnten bis zu Konzentrationen von 10 *µ*M wachsen.

Einer der aus H.trapanicum hervorgegangenen Stämme war der Mikroorganismus Halobacterium CB1 (DSM 11147).

Halobacterium CB1 wurde zur weiteren Adaption an spezielle Verbindungen, wie Trichlorphenole, DDT oder Lindan einem weiteren Selektionsprozeß unterzogen. Hierzu wurde CB1 in Flüssigkultur (Fermentationsbedingungen wie in Beispiel 2 beschrieben) in Gegenwart einer Konzentration von 1 *µ*M des jeweiligen Giftstoffes angezogen und überimpft, bis die finale Zelldichte der Kultur die Zelldichte einer Vergleichskultur erreicht hatte, die in Abwesenheit des Giftstoffs angezogen worden war. Anschließend wurde die Konzentration des Giftstoffs auf 10 *µ*M erhöht. Dieser Prozeß wurde entsprechend wiederholt, bis die maximal tolerable Konzentration oder die Löslichkeitsgrenze des Giftstoffs erreicht war.

Dabei wurden Abkömmlinge von CB1 erhalten, die an spezielle Verbindungen adaptiert sind. So ist CB1-H an 2,3,4-Trichlorphenol bis zu einer Maximalkonzentration von 50 *µ*M adaptiert. Die Stämme CB1-G und CB1-K sind an Lindan bzw. DDT bis zur Löslichkeitsgrenze dieser Giftstoffe im Kulturmedium adaptiert. Die Stämme CB1-N und CB1-0 sind an 2-Chlordibenzodioxin bzw. 1,2,3,4-Tetrachlordibenzodioxin bis zu einer Maximalkonzentration > 100 *µ*M adaptiert.

Auch Isolate der Gattungen Haloarcula und Haloferax konnten an Lindan, DDT und Dioxine im Kulturmedium adaptiert werden.

Einer der aus Haloferax mediterranei hervorgegangenen Stämme war der Mikororganismus Hf-X1 (DSM 11795), der besonders zum Abbau von Tri-, Tetra- und Pentachlorphenolen geeignet ist.

Einer der aus Haloarcula californiae hervorgegangenen Stämme war der Mikroorganismus Hc-U2 (DSM 11794), der besonders geeignet zum Abbau bromierter Kohlenwasserstoffe ist.

### Beispiel 2

### Abbau von Trichlorphenolen

Die Zellen wurden als 35 ml Kulturen in 100 ml Erlenmeyerkolben, angeimpft mit 1 ml Impfkultur, in einem geeigneten Wachstumsmedium (250 g NaCl, 20 g Mg SO₄ · 7 H₂O, 3 g Trinatriumcitrat, 2 g KCl, 10 g Oxid bacteriologisches Pepton, 1 l H₂O) bei 40 °C unter aeroben oder mikroaeroben Bedingungen kultiviert. Dem Kolben wurden die Giftstoffe 2,3,4-, 2,3,5- oder 2,4,5-Trichlorphenol, gelöst in geringen Mengen Ethanol oder Isopropanol, zu Beginn der Fermentation zugesetzt. Die Endkonzentration der Giftstoffe war 50 *µ*M. Nach 7-10 Tagen Inkubation bei 40 °C auf einem Schüttler mit 100 Upm wurde die gesamte Kulturbrühe mit 2 x 20 ml einer Lösung von 5 % Essigsäure in Essigsäureethylester extrahiert. Der Kolben wurde nochmals mit der gleichen Extraktionslösung gespült.

Die vereinigten organischen Extrakte wurden gegebenenfalls nach Einengen im Stickstoffstrom mittels kombinierter Gaschromatographie/ei-Massenspektrometrie analysiert. Weiterhin wurden während der Fermentation Kontrollproben von jeweils 600 *µ*l Kulturbrühe mit 800 *µ*l Extraktionslösung extrahiert und analysiert. Parallel hierzu wurden negative Kontrollen (Inkubation des Giftstoffs im Medium in Abwesenheit von Zellen) durchgeführt, bei denen kein signifikanter Abbau des Giftstoffes gefunden wurde.

Zur GC-MS-Analyse wurde ein 1 *µ*l des Essigesterestrakts über ein Kaltaufgabesystem (Gerstel KAS-502) direkt auf eine semipolare 30 m DP-1-Säule (J & W Scientific Inc.) aufgespritzt und über einen Gradienten (80-250 °C, 10 °C/Min) chromatographiert. Der Nachweis erfolgte "splitless" mit einem Finnigan MAT312 Elektronenaufprall-Massenspektrometer.

Bei Fermentation des an Trichlorphenole adaptierten Mikroorganismus Halobacterium CB1-H konnten nach einer Inkubationsdauer von 7-8 Tagen unter aeroben Bedingungen keine chlorierten organischen Substanzen mehr nachgewiesen werden. Unter mikroaeroben Bedingungen wurde ein langsamerer Abbau gefunden.

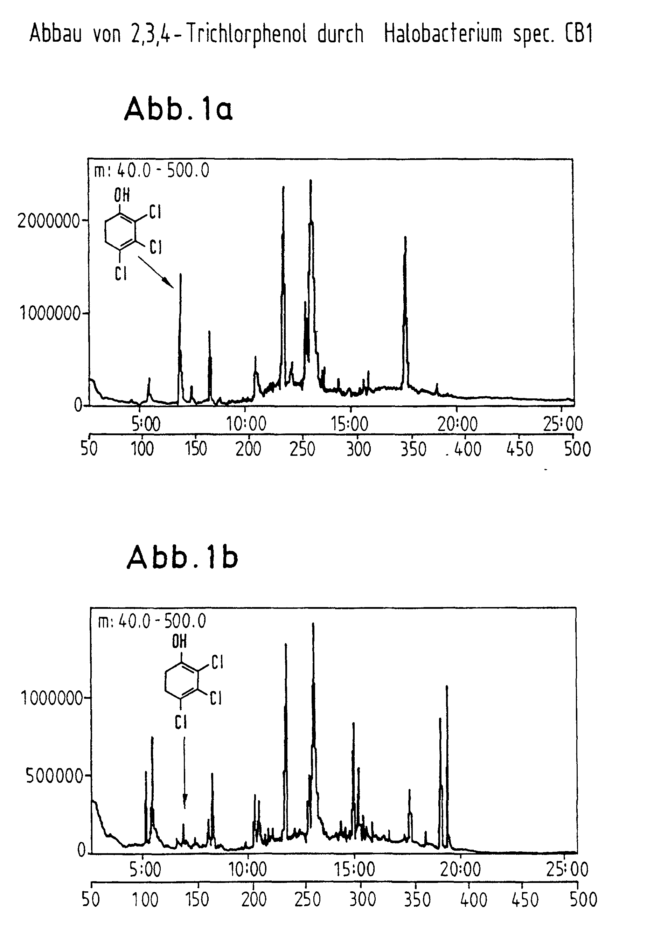

Abb. 1a zeigt ein Gaschromatogram des organischen Extrakts einer Fermentation von Halobacterium CB1-H in Gegenwart von 2,3,4-Trichlorphenol (10 *µ*M) nach 0 Tagen. Aus Abb. 1b ist ersichtlich, daß nach 5-tägiger Inkubation unter mikroaeroben Bedingungen ein fast vollständiger Abbau von 2,3,4-Trichlorphenol stattgefunden hat.

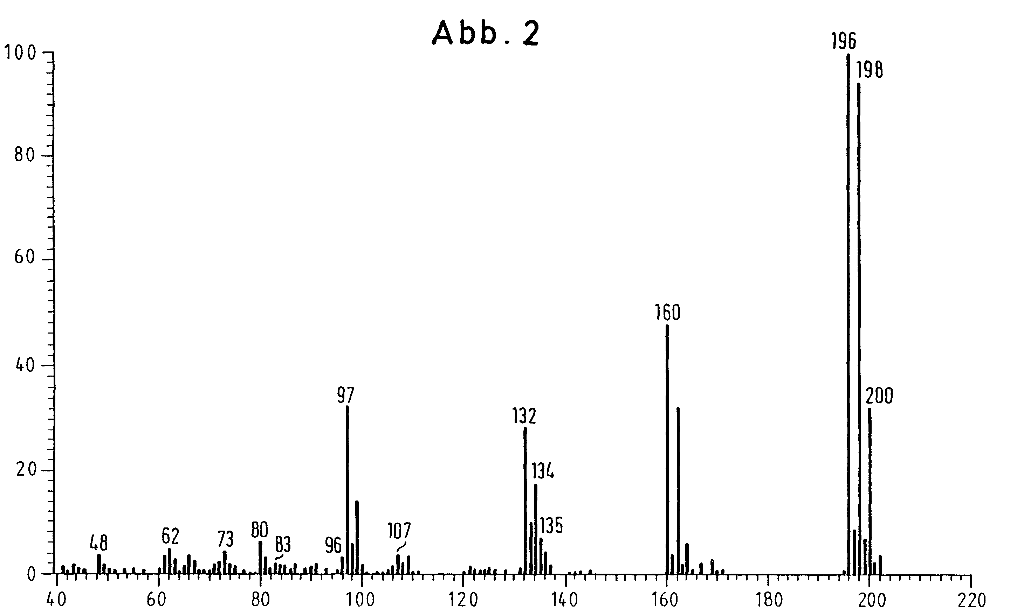

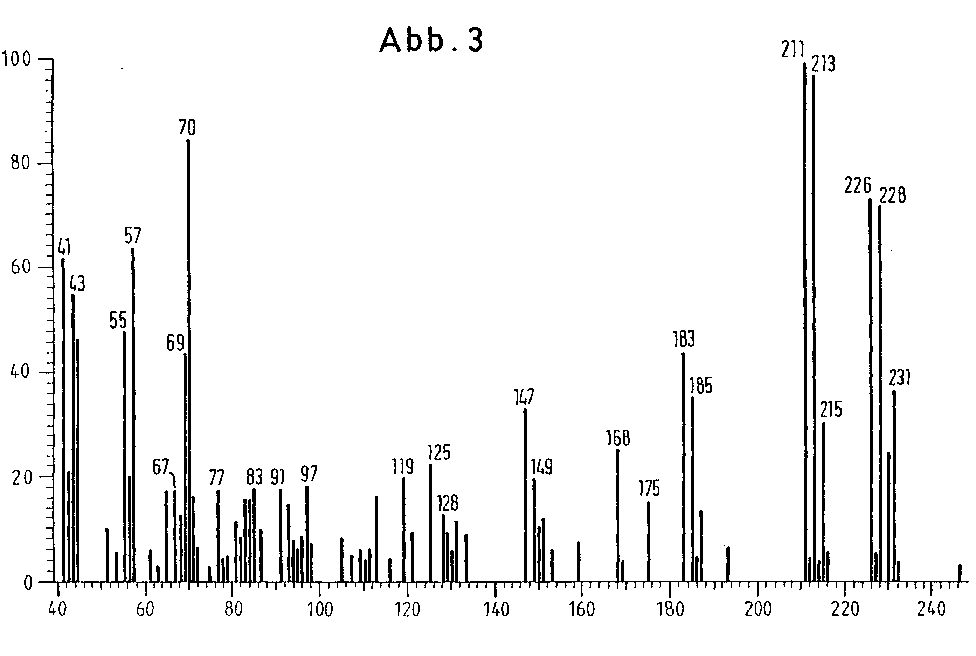

Abb. 2 zeigt das Massenspektrum von 2,3,4-Trichlorphenol (m/e = 196/198/200) und Abb. 3 das Massenspektrum eines unter mikroaeroben Bedingungen identifizierten mutmaßlichen Metaboliten, nämlich 3,4,5-Trichlor-O-methylbrenzcatechin (m/e = 226/228/230).

### Beispiel 3

### Abbau von Lindan

Der Abbau von Lindan wurde mit dem Organismus Halobacterium CB1-G in Gegenwart von 1 mM Lindan durchgeführt. Die Herstellung und Analyse von Fermentationsextrakten erfolgte wie in Beispiel 2 beschrieben.

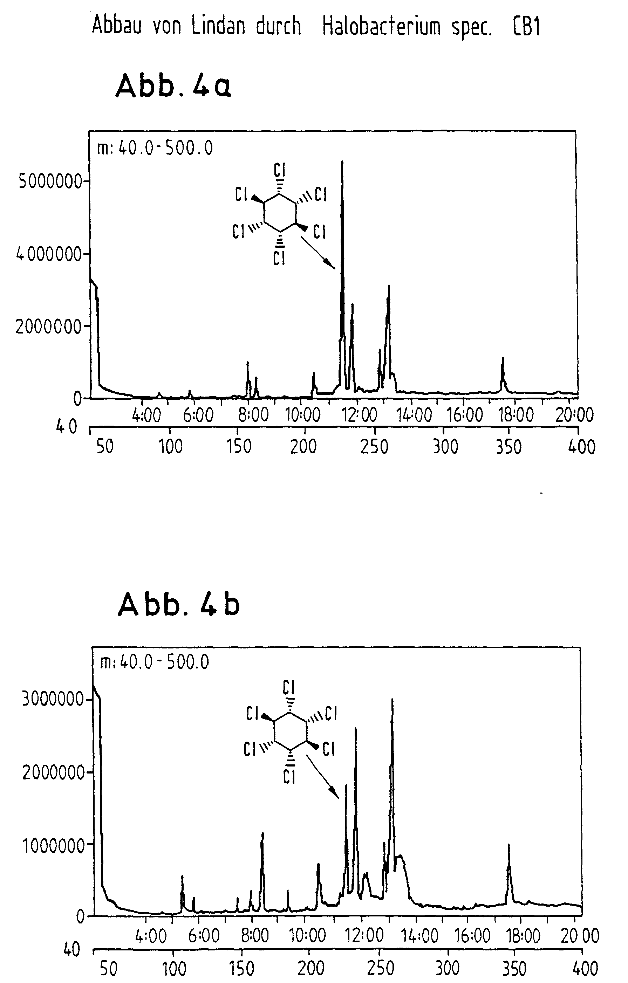

Abb. 4a und 4b zeigen Gaschromatogramme der organischen Extrakte einer Fermentation in Gegenwart von 1 mM Lindan nach 2 bzw. 15 Tagen unter aeroben Bedingungen. Es ist eine deutliche Verringerung des Lindan-Peaks zu erkennen.

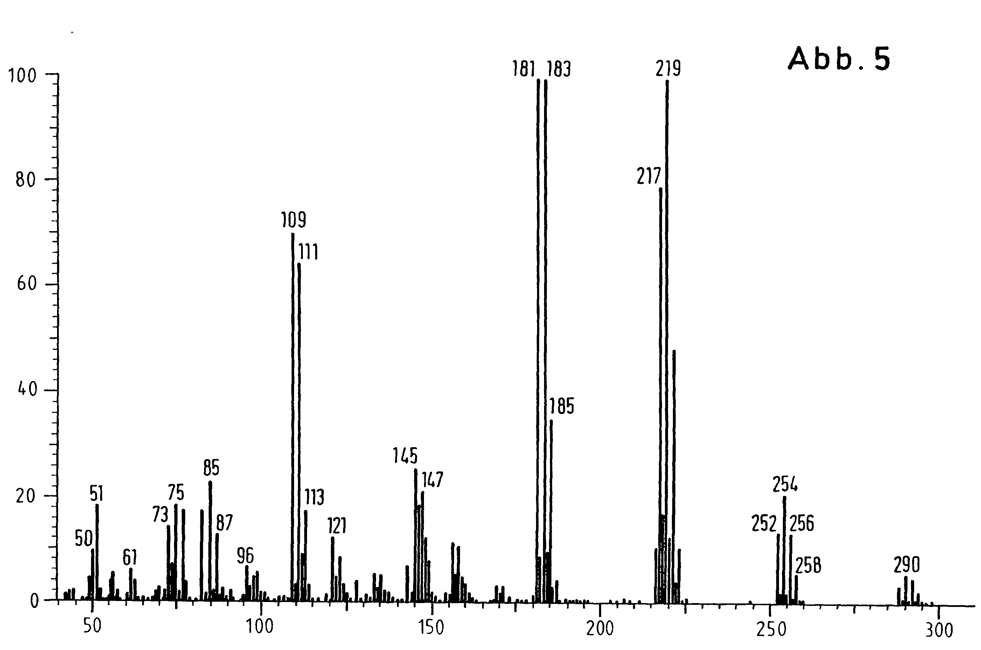

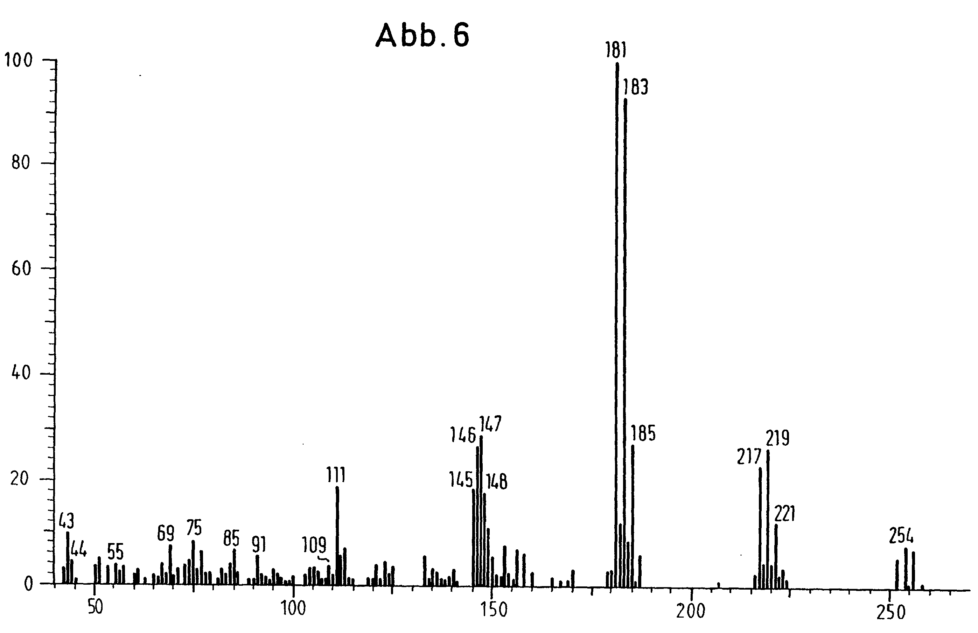

Abb. 5 zeigt das Massenspektrum von Lindan (m/e = 288/290/292). Abb. 6 zeigt das Massenspektrum eines unter mikroaeroben Bedingungen nachweisbaren mutmaßlichen Metaboliten von Lindan, nämlich 1,3,4,5,6-Pentachlorcyclohexen (m/e = 252/254/256).

### Beispiel 4

### Abbau von DDT

Der Abbau von 2,2-Bis(4-chlorphenyl)-1,1,1-trichlorethan (DDT) wurde durch Fermentation mit dem Organismus Halobacterium CB1-K in Gegenwart von 1 mM DDT wie in Beispiel 2 beschrieben durchgeführt. Auch die Herstellung der Extrakte und die Analytik erfolgte wie in Beispiel 2 beschrieben.

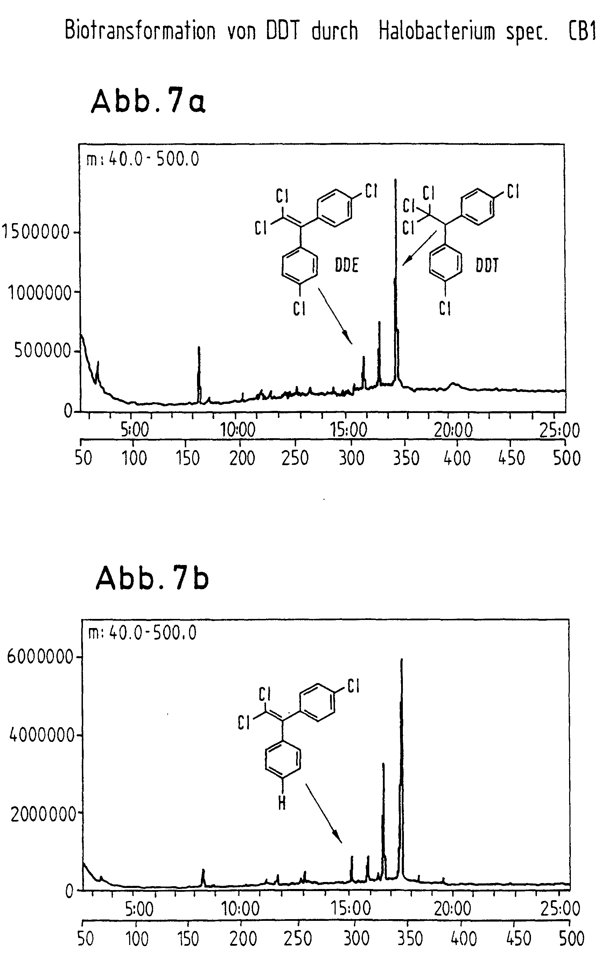

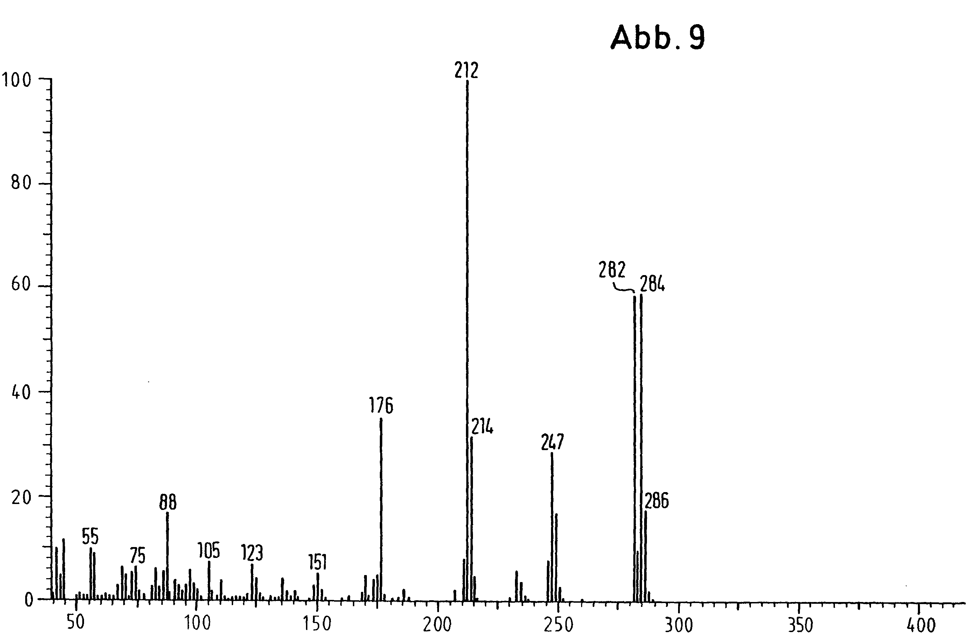

Abb. 7a und 7b zeigen Gaschromatogramme der organischen Extrakte einer Fermentation in Gegenwart von 1 mM DDT nach 2 bzw. 8 Tagen. Neben dem Peak von DDT ist in Abb. 7a auch der Peak des Metaboliten 2,2-Bis-(4-chlorphenyl)-1,1-dichlorethylen (DDE) zu erkennen. In Abb. 7b ist noch ein weiterer Metabolit (vgl. Abb. 9) zu erkennen.

Das in Abb. 7a gezeigte Chromatogramm stammt aus einem Extrakt, der aus analytischen Gründen (zu große DDT-Konzentration) 10-fach verdünnt wurde. Aus einem Vergleich der Chromatogramme in Abb. 7a und 7b geht hervor, daß mit zunehmender Inkubationsdauer die DDT-Konzentration im Extrakt abnimmt und ein verändertes Metabolitenspektrum auftritt.

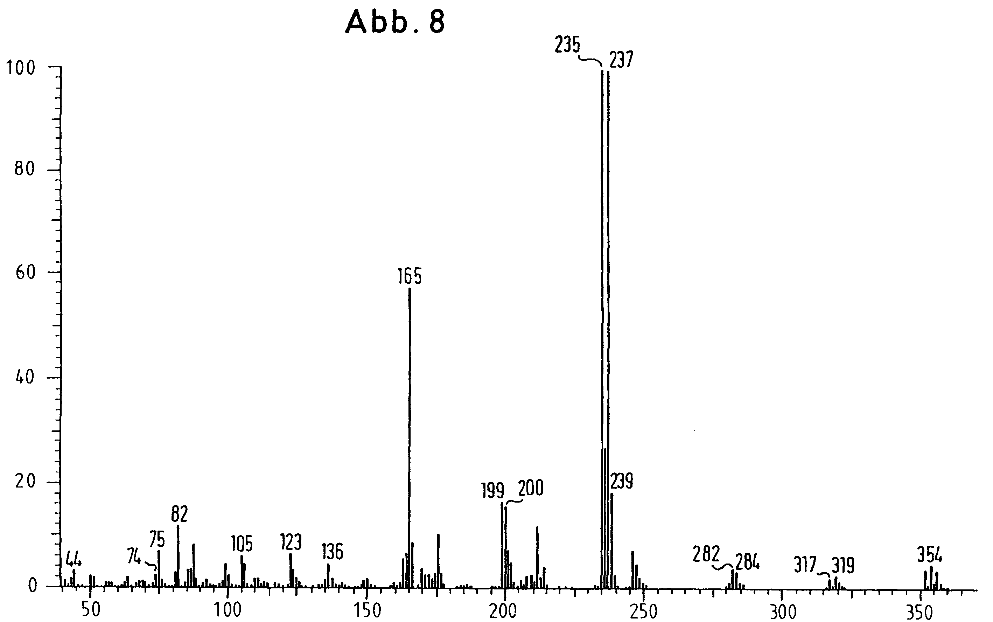

Abb. 8 zeigt das Massenspektrum von DDT (m/e = 352/354/356). Abb. 9 zeigt das Massenspektrum eines bei mikroaerober Fermentation auftretenden weiteren mutmaßlichen Metaboliten, nämlich 2-(4-Chlorphenyl)-2-phenyl-1,1-dichlorethylen (m/e = 282/284/286).

### Beispiel 5

Analog den in den Beispielen 2 bis 4 beschriebenen Verfahren wurden weitere organische Verbindungen und Mikroorganismenstämme getestet.

Die Ergebnisse waren wie folgt:

Stämme hervorgegangen aus Halobacterium CB1 (DSM 11147) und Haloferax mediterranei waren besonders für den Abbau von 2,3,6-Trichlorphenol, 2,4,6-Trihalogenphenol (Halogen = Fluor, Chlor, Brom, Jod), 2,4,5-Trichlorphenol, 2,3,4-Trichlorphenol, 2,3,4-Trifluorphenol, 2,3,5-Trichlorphenol, 2,3,5-Trifluorphenol und 3,4,5-Trichlorphenol geeignet.

Stämme hervorgegangen aus Halobacterium CB1 waren besonders geeignet zum Abbau von Lindan.

Stämme hervorgegangen aus Haloarcula hispanica und Haloferax gibbonsii waren besonders geeignet zum Abbau von DDT, DDE und DDD.

Stämme hervorgegangen aus Haloferax volcanii waren besonders geeignet zum Abbau von Chlorparaffinfraktionen.

Stämme hervorgegangen aus Haloarcula californiae und Halorubrum saccharovorum waren besonders geeignet zum Abbau bromierter Kohlenwasserstoffe wie etwa Decabromdiphenylether und Bis-2,2-(3,5-dibrom-4-hydroxyphenyl)-propan.

Stämme hervorgegangen aus Haloferax mediterranei und Halobacterium CB1 waren - insbesondere unter mikroaeroben Fermentationsbedingungen - für den Abbau von Pentahalogenphenol (Halogen = Chlor, Fluor, Brom) geeignet.

Stämme hervorgegangen aus Haloferax mediterranei waren besonders zum Abbau von 2,3,4,5-Tetrachlorphenol und 2,3,4,6-Tetrachlorphenol geeignet.

## Patentansprüche

1. Verfahren zum zumindest partiellen Abbau von halogenierten organischen Verbindungen, dadurch gekennzeichnet, daß man die Verbindungen mit halophilen Archaea oder mit Extrakten oder Bestandteilen davon in Kontakt bringt und unter Bedingungen inkubiert, bei denen ein Abbau der organischen Verbindungen stattfindet.

2. Verfahren nach Anspruch 1, dadurch gekennzeichnet, daß die halophilen Archaea ausgewählt werden aus Organismen der Gattungen Haloarcula, Halobacterium, Halococcus, Haloferax, Halorubrum, Natronobacterium und Natronococcus.

3. Verfahren nach Anspruch 2, dadurch gekennzeichnet, daß die halophilen Archaea ausgewählt werden aus Organismen der Gattung Haloarcula, Halobacterium, Haloferax und Halorubrum.

4. Verfahren nach Anspruch 3, dadurch gekennzeichnet, daß man Organismen aus der Gattung Halobacterium verwendet.

5. Verfahren nach einem der Ansprüche 1-4, dadurch gekennzeichnet, daß man Organismen verwendet, die einem Selektionsprozeß zur Adaption an halogenierte organische Verbindungen unterworfen worden sind.

6. Verfahren nach Anspruch 5, dadurch gekennzeichnet, daß man einen Organismus ausgewählt aus Halobacterium CB1 (DSM 11147), Haloferax Hf-X1 (DSM 11795) und Haloarcula Hc-U2 (DSM 11794), oder einen an spezielle Verbindungen adaptierten Abkömmling davon verwendet.

7. Verfahren nach einem der Ansprüche 1-6, dadurch gekennzeichnet, daß man einen Organismus verwendet, der eine Toleranz gegenüber den abzubauenden Verbindungen in einer Konzentration von mindestens 10 *µ*M im Medium aufweist.

8. Verfahren nach Anspruch 7, dadurch gekennzeichnet, daß man einen Organismus verwendet, der eine Toleranz gegenüber den abzubauenden Verbindungen in einer Konzentration von mindestens 100 *µ*M im Medium aufweist.

9. Verfahren nach einem der Ansprüche 1-8, dadurch gekennzeichnet, daß die abzubauenden Verbindungen ausgewählt werden aus organischen Verbindungen, die Kohlenstoff, gegebenenfalls Wasserstoff und Heteroatome sowie mindestens ein Halogenatom enthalten.

10. Verfahren nach Anspruch 9, dadurch gekennzeichnet, daß die abzubauenden Verbindungen mindestens ein Chloratom enthalten.

11. Verfahren nach Anspruch 9 oder 10, dadurch gekennzeichnet, daß die Halogenatome an aliphatische, aromatische oder/und olefinische Kohlenstoffatome gebunden sind.

12. Verfahren nach einem der Ansprüche 9-11, dadurch gekennzeichnet, daß die abzubauenden Verbindungen aus gegebenenfalls mehrfach halogierten aromatischen Verbindungen ausgewählt werden.

13. Verfahren nach Anspruch 12, dadurch gekennzeichnet, daß die abzubauenden Verbindungen aus halogierten Phenolen, Benzolen, Dioxinen und Furanen ausgewählt werden.

14. Verfahren nach Anspruch 13, dadurch gekennzeichnet, daß man 2,4,6-Trihalogenphenol (Halogen = Fluor, Chlor, Brom Jod), 2,3,4-Trichlorphenol, 2,3,4-Trifluorphenol, 2,3,5-Trichlorphenol, 2,3,6-Trichlorphenol, 2,4,5-Trichlorphenol, 3,4,5-Trichlorphenol, 2,3,4,5-Tetrachlorphenol, 2,3,4,6-Tetrachlorphenol oder/und Pentahalogenphenol (Halogen = Fluor, Chlor, Brom) abbaut.

15. Verfahren nach Anspruch 13, dadurch gekennzeichnet, daß man 2-Chlordibenzodioxin (2-MCDD) oder/und 1,2,3,4-Tetrachlordibenzodioxin (1,2,3,4-TCDD) abbaut.

16. Verfahren nach Anspruch 12, dadurch gekennzeichnet, daß man DDT, DDE oder/und DDD abbaut.

17. Verfahren nach Anspruch 11, dadurch gekennzeichnet, daß man Lindan (γ-Hexachlorcyclohexan) abbaut.

18. Verfahren nach einem der Ansprüche 1 bis 17, dadurch gekennzeichnet, daß man den Abbau in Gegenwart lösungsvermittelnder Substanzen durchführt.

19. Verfahren nach Anspruch 18, dadurch gekennzeichnet, daß die lösungsvermittelnden Substanzen aus Cyclodextrinen, Alkylglycosiden und Cholaten ausgewählt werden.

20. Verfahren nach Anspruch 18 oder 19, dadurch gekennzeichnet, daß man die lösungsvermittelnden Substanzen in einer Menge von mindestens 0,01 Moläquivalent pro Mol der abzubauenden Verbindungen verwendet.

21. Verfahren zur Herstellung von an halogenierte organische Verbindungen adaptierten halophilen Archaea,
**dadurch gekennzeichnet,**
daß man einen Ausgangsorganismus auswählt, der eine Toleranz gegenüber einer vorbestimmten Konzentration einer oder mehrerer ausgewählter halogenierter organischer Verbindungen aufweist, und diesen Ausgangsorganismus einem Selektionsprozeß unterwirft, wobei ein adaptierter Organismus erhalten wird, der gegenüber dem Ausgangsorganismus eine erhöhte Toleranz oder/und Abbauleistung bezüglich den ausgewählten Verbindungen aufweist.

22. Verfahren nach Anspruch 21, dadurch gekennzeichnet, daß der Selektionsprozeß das Ausplattieren des Ausgangsorganismus auf einem festen Kulturmedium in Gegenwart von halogenierten organischen Verbindungen, das Identifizieren eines bei der gewählten Giftstoffkonzentration noch wachsenden Organismus und gegebenenfalls ein ein- oder mehrmaliges Wiederholen dieser Prozedur bei steigenden Giftstoffkonzentrationen umfaßt.

23. Verfahren nach Anspruch 21 oder 22, dadurch gekennzeichnet, daß man als adaptierten Organismus Halobacterium CB1 (DSM 11147) oder einen Organismus mit mindestens gleicher Toleranz oder/und Abbauleistung erhält.

24. Verfahren nach einem der Ansprüche 21-23, dadurch gekennzeichnet, daß man den adaptierten Organismus einem weiteren Selektionsprozeß zur Erhöhung der Toleranz oder/und Abbauleistung bezüglich spezieller halogenierter organischer Verbindungen unterwirft.

25. Halophiler Archaeaorganismus, der an halogenierte organische Verbindungen adaptiert ist, erhältlich durch ein Verfahren nach einem der Ansprüche 21-24.

26. Organismus nach Anspruch 25, dadurch gekennzeichnet, daß er Halobacterium CB1 (DSM 11147) oder ein Organismus mit mindestens gleicher Toleranz oder/und Abbauleistung bezüglich halogenierten organischen Verbindungen ist.

27. Organismus nach Anspruch 25, dadurch gekennzeichnet, daß er Haloferax Hf-X1 (DSM 11795) oder ein Organismus mit mindestens gleicher Toleranz oder/und Abbauleistung bezüglich halogenierten organischen Verbindungen ist.

28. Organismus nach Anspruch 25, dadurch gekennzeichnet, daß er Haloarcula Hc-U2 (DSM 11794) oder ein Organismus mit mindestens gleicher Toleranz oder/und Abbauleistung bezüglich halogenierten organischen Verbindungen ist.

## Claims

1. Process for the at least partial degradation of halogenated organic compounds, wherein the compounds are brought into contact with halophilic Archaea or with extracts or components thereof and are incubated under conditions under which a degradation of the organic compounds takes place.

2. Process as claimed in claim 1, wherein the halophilic Archaea are selected from organisms of the genera Haloarcula, Halobacterium, Halococcus, Haloferax, Halorubrum, Natronobacterium and Natronococcus.

3. Process as claimed in claim 2, wherein the halophilic Archaea are selected from organisms of the genus Haloarcula, Halobacterium, Haloferax and Halorubrum.

4. Process as claimed in claim 3, wherein organisms of the genus Halobacterium are used.

5. Process as claimed in one of the claims 1-4, wherein organisms are used which have been subjected to a selection process to adapt them to halogenated organic compounds.

6. Process as claimed in claim 5, wherein an organism selected from Halobacterium CB1 (DSM 11147), Haloferax Hf-X1 (DSM 11795) and Haloarcula Hc-U2 (DSM 11794) or a descendant thereof adapted to special compounds is used.

7. Process as claimed in one of the claims 1-6, wherein an organism is used which tolerates the compounds to be degraded at a concentration of at least 10 *µ*M in the medium.

8. Process as claimed in claim 7, wherein an organism is used which has a tolerance towards the compounds to be degraded at a concentration of at least 100 *µ*M in the medium.

9. Process as claimed in one of the claims 1-8, wherein the compounds to be degraded are selected from organic compounds which contain carbon, optionally hydrogen and heteroatoms and at least one halogen atom.

10. Process as claimed in claim 9, wherein the compounds to be degraded contain at least one chlorine atom.

11. Process as claimed in claims 9 or 10, wherein the halogen atoms are bound to aliphatic, aromatic or/and olefinic carbon atoms.

12. Process as claimed in one of the claims 9-11, wherein the compounds to be degraded are selected from optionally polyhalogenated aromatic compounds.

13. Process as claimed in claim 12, wherein the compounds to be degraded are selected from halogenated phenols, benzenes, dioxins and furans.

14. Process as claimed in claim 13, wherein 2,4,6-trihalogenphenol (halogen = fluorine, chlorine, bromine, iodine), 2,3,4-trichlorophenol, 2,3,4-trifluorophenol, 2,3,5-trichlorophenol, 2,3,6-trichlorophenol, 2,4,5-trichlorophenol, 3,4,5-trichlorophenol, 2,3,4,5-tetrachlorophenol, 2,3,4,6-tetrachlorophenol or/and pentahalogenphenol (halogen = fluorine, chlorine, bromine) is degraded.

15. Process as claimed in claim 13, wherein 2-chlorodibenzodioxin (2-MCDD) or/and 1,2,3,4-tetrachlorodibenzodioxin (1,2,3,4-TCDD) is degraded.

16. Process as claimed in claim 12, wherein DDT, DDE or/and DDD is degraded.

17. Process as claimed in claim 11, wherein lindane (γ-hexachlorocyclohexane) is degraded.

18. Process as claimed in one of the claims 1 to 17, wherein the degradation is carried out in the presence of solubilizing substances.

19. Process as claimed in claim 18, wherein the solubilizing substances are selected from cyclodextrins, alkylglycosides and cholates.

20. Process as claimed in claims 18 or 19, wherein the solubilizing substances are used in an amount of at least 0.01 mole equivalents per mole of the compounds to be degraded.

21. Process for the production of halophilic Archaea adapted to halogenated organic compounds,
**wherein**
a starting organism is selected which has a tolerance towards a predetermined concentration of one or several selected halogenated organic compounds and this starting organism is subjected to a selection process in which an adapted organism is obtained which has an increased tolerance or/and degradation rate with respect to the selected compounds compared to the starting organism.

22. Process as claimed in claim 21, wherein the selection process comprises plating out the starting organism on a solid culture medium in the presence of halogenated organic compounds, identifying an organism which still grows under the selected concentration of the toxic substance and optionally repeating this procedure once or several times with increasing concentrations of the toxic substance.

23. Process as claimed in claims 21 or 22, wherein Halobacterium CB1 (DSM 11147) or an organism with an at least equivalent tolerance or/and degradation rate is obtained as the adapted organism.

24. Process as claimed in one of the claims 21-23, wherein the adapted organism is subjected to an additional selection process to increase the tolerance or/and degradation rate with respect to special halogenated organic compounds.

25. Halophilic Archaea organism which is adapted to halogenated organic compounds obtainable by a process as claimed in one of the claims 21-24.

26. Organism as claimed in claim 25, wherein it is Halobacterium CB1 (DSM 11147) or an organism with an at least equivalent tolerance or/and degradation rate with respect to halogenated organic compounds.

27. Organism as claimed in claim 25, wherein it is Haloferax Hf-X1 (DSM 11795) or an organism with an at least equivalent tolerance or/and degradation rate with respect to halogenated organic compounds.

28. Organism as claimed in claim 25, wherein it is Haloarcula Hc-U2 (DSM 11794) or an organism with an at least equivalent tolerance or/and degradation rate with respect to halogenated organic compounds.

## Revendications

1. Procédé pour la décomposition au moins partielle de composés organiques halogénés, caractérisé en ce que l'on met en contact les composés avec des archaea ou archéobactéries halophiles ou avec des extraits ou constituants de celles-ci et que l'on incube dans des conditions où se produit une décomposition des composés organiques.

2. Procédé selon la revendication 1, caractérisé en ce que les archaea ou archéobactéries halophiles sont choisies à partir des organismes des espèces haloarcula, halobacterium, halococcus, haloferax, halorubrum, natronobacterium et natronococcus.

3. Procédé selon la revendication 2, caractérisé en ce que les archaea ou archéobactéries halophiles sont choisies à partir de l'espèce haloarcula, halobacterium, haloferax et halorubrum.

4. Procédé selon la revendication 3, caractérisé en ce que l'on utilise des organismes de l'espèce halobacterium.

5. Procédé selon l'une des revendications 1-4, caractérisé en ce que l'on utilise des organismes qui ont été soumis à un processus de sélection pour l'adaptation à des composés organiques halogénés.

6. Procédé selon la revendication 5, caractérisé en ce que l'on utilise un organisme choisi parmi halobacterium CB1 (DSM 11147), haloferax Hf-X1 (DSM 11795) et haloarcula hc-U2 (DSM 11794) ou un dérivé de ceux-ci adapté à des composés spéciaux.

7. Procédé selon l'une des revendications 1-6, caractérisé en ce que l'on utilise un organisme qui présente une tolérance vis-à-vis des composés à décomposer dans une concentration d'au moins 10 µM dans le milieu.

8. Procédé selon la revendication 7, caractérisé en ce que l'on utilise un organisme qui présente une tolérance vis-à-vis des composés à décomposer dans une concentration d'au moins 100 µM dans le milieu.

9. Procédé selon l'une des revendications 1-8, caractérisé en ce que les composés à décomposer sont choisis à partir de composés organiques qui contiennent du carbone, éventuellement de l'hydrogène et des hétéroatomes ainsi qu'au moins un atome d'halogène.

10. Procédé selon la revendication 9, caractérisé en ce que les compositions à décomposer contiennent au moins un atome de chlore.

11. Procédé selon la revendication 9 ou 10, caractérisé en ce que les atomes d'halogène sont fixés sur des atomes de carbone aliphatiques, aromatiques ou/et oléfiniques.

12. Procédé selon l'une des revendications 9-11,
caractérisé en ce que les composés à décomposer sont choisis à partir de composés aromatiques, éventuellement halogénés plusieurs fois.

13. Procédé selon la revendication 12, caractérisé en ce que les composés à décomposer sont choisis à partir de phénols, benzènes, dioxines et furannes halogénés.

14. Procédé selon la revendication 13, caractérisé en ce que l'on décompose le 2,4,6-trihalogénophénol (halogène = fluor, chlore, brome, iode), le 2,3,4-trichlorophénol, le 2,3,4-trifluorophénol, le 2,3,5-trichlorophénol, le 2,3,6-trichlorophénol, le 2,4,5-trichlorophénol, le 3,4,5-trichlorophénol, le 2,3,4,5-tétrachlorophénol, le 2,3,4,6-tétrachlorophénol ou/et le pentahalogénophénol (halogène = fluor, chlore, brome) .

15. Procédé selon la revendication 13, caractérisé en ce que l'on décompose la 2-chlorodibenzodioxine (2-MCDD) ou/et la 1,2,3,4-tétrachlorodibenzodioxine (1,2,3,4-TCDD).

16. Procédé selon la revendication 12, caractérisé en ce que l'on décompose DDT, DDE ou/et DDD.

17. , Procédé selon la revendication 11, caractérisé en ce que l'on décompose le lindan (γ-hexachlorocyclohexane).

18. Procédé selon l'une des revendications 1 à 17,
caractérisé en ce que l'on effectue la décomposition en présence de substances de solubilisation.

19. Procédé selon la revendication 18, caractérisé en ce que l'on choisit les substances de solubilisation parmi les cyclodextrines, alkylglycosides et cholates.

20. Procédé selon la revendication 18 ou 19, caractérisé en ce que l'on utilise les substances de solubilisation dans une quantité d'au moins 0,01 équivalent molaire par mole des composés à décomposer.

21. Procédé pour la préparation d'archaea ou archéobactéries halophiles adaptées à des compositions organiques halogénées,
caractérisé en ce que
l'on choisit un organisme de départ qui présente une tolérance vis-à-vis d'une concentration prédéterminée d'un ou de plusieurs composés organiques halogénés sélectionnés et en ce que l'on soumet cet organisme de départ à un processus de sélection, moyennant quoi on obtient un organisme adapté qui présente une tolérance accrue vis-à-vis de l'organisme de départ ou/et une capacité de décomposition accrue vis-à-vis des composés choisis.

22. Procédé selon la revendication 21, caractérisé en ce que le processus de sélection comprend l'étalement de l'organisme de départ sur un milieu de culture solide en présence de composés organiques halogénés, l'identification d'un organisme encore en croissance dans la concentration toxique choisie et éventuellement, une répétition une seule ou plusieurs fois de ce processus avec des concentrations toxiques croissantes.

23. Procédé selon la revendication 21 ou 22, caractérisé en ce qu'en tant qu'organisme adapté, on obtient un halobacterium CB1 (DSM 11147) ou un organisme ayant au moins la même tolérance ou/et capacité de décomposition.

24. Procédé selon l'une des revendications 21-23,
caractérisé en ce que l'on soumet l'organisme adapté à un autre processus de sélection pour accroître la tolérance ou/et la capacité de décomposition à l'égard des composés organiques halogénés spéciaux.

25. Archéoorganisme halophile qui est adapté à des composés organiques halogénés et pouvant être obtenu par un procédé selon l'une des revendications 21-24.

26. Organisme selon la revendication 25, caractérisé en ce qu'il s'agit d'un halobacterium CB1 (DSM 11147) ou d'un organisme ayant au moins la même tolérance ou/et capacité de décomposition à l'égard des composés organiques halogénés.

27. Organisme selon la revendication 25, caractérisé en ce qu'il s'agit d'un haloferax Hf-X1 (DSM 11795) ou d'un organisme ayant au moins la même tolérance ou/et capacité de décomposition à l'égard des composés organiques halogénés.

28. Organisme selon la revendication 25, caractérisé en ce qu'il s'agit d'haloarcula Hc-U2 (DSM 11794) ou d'un organisme ayant au moins la même tolérance ou/et capacité de décomposition à l'égard des composés organiques halogénés.
